# EUROPÄISCHE PATENTSCHRIFT

(11) **EP 1 269 925 B1**
(45) Veröffentlichungstag und Bekanntmachung des Hinweises auf die Patenterteilung: **17.12.2003**
(21) Anmeldenummer: 01114904.4
(22) Anmeldetag: 19.06.2001
(51) Int. Cl.: A61B 17/34, A61M 1/00

(54) **Zugangskanüle für endoskopische Operationen, insbesondere für die Arthroskopie**
Access cannula for endoscopic surgery, especially for arthroscopy
Canule d'accès pour une opération endoscopique, notamment pour l'arthroscopie

(43) Veröffentlichungstag der Anmeldung: 02.01.2003
(73) Patentinhaber: Karl Storz GmbH & Co. KG, 78532 Tuttlingen (DE)
(72) Erfinder: Lajtai, Georg Dr., 4600 Wels (AT); Sauer, Michael, Dipl.-Ing., 78532 Tuttlingen (DE); Timmermans, André, 7261 JH Ruurlo (NL)
(74) Vertreter: Weller, Wolfgang, Dr.

(56) Entgegenhaltungen:
- WO-A-01/08563
- US-A- 5 234 455
- US-A- 5 364 372
- US-A- 5 800 451
- US-A- 5 957 888

## Beschreibung

Die Erfindung betrifft eine Zugangskanüle für endoskopische Operationen, insbesondere für die Arthroskopie, mit einem Kanülenrohr und mit einem Ventilkörper.

Ein derartiges Kanülenrohr ist aus der WO-A-01/08563 bekannt.

Bei minimal-invasiven Operationstechniken, die sich immer weiter verbreiten, wird ein solcher Zugang über einen Trokar bewerkstelligt. Ein Trokar weist einen hülsen- oder kanülenförmigen, hohlrohrförmigen Körper auf, der in den Körper eingebracht wird, beispielsweise durch die abdominale Wand hindurch, um im inneren Bauchraum Operationen durchzuführen. Durch den Trokar werden dann entsprechende Instrumente und insbesondere Endoskope hindurchgeführt, um die im Inneren des Körpers durchgeführte Operation visuell beobachten zu können.

Zum Setzen des Trokars wird in die Trokarhülse ein angespitzter Trokardorn bzw. Mandrin eingesetzt und eine Hautincision geschaffen, um die Zugangskanüle, also hier die Trokarhülse, in den Körper einzubringen.

Bei minimal-invasiven Operationen an Weichteilen, wie beispielsweise durch die Bauchwand, kann der Trokar gleich in der notwendigen Größe eingebracht werden.

Weit schwieriger ist, einen Zugang bei arthroskopischen Vorgängen zu schaffen. Will man eine minimal-invasive endoskopische Operation in einem Gelenk durchführen, sind die Räume zwischen zwei ein Gelenk bildenden Knochen oftmals sehr gering, so daß ein Trokar mittels der zuvor beschriebenen Technik nicht einfach gesetzt werden kann.

Oftmals ist es erwünscht oder auch notwendig, im Operationsfeld mehrere Zugangskanülen zu setzen, um das Operationsfeld entweder aus mehreren Richtungen zu beobachten, oder um aus mehreren Richtungen einen Zugang zu dem Gelenk zu erhalten.

Ein weiteres Problem besteht darin, daß zur besseren Sicht an der Operationsstelle durch die Zugangskanüle Gase und/oder Flüssigkeiten zugeführt werden, um den Körper im Operationsfeld durch Überdruck etwas aufzudehnen. Durch die Zugangskanüle werden auch oftmals Spülflüssigkeiten hindurchgeführt. Um ein Auslaufen von Körperflüssigkeiten, von Gasen oder von Spülflüssigkeiten aus dem proximalen Ende der Zugangskanüle zu verhindern, ist dort üblicherweise ein Ventil vorhanden, das einerseits für einen dichten Abschluß des Kanülenrohres sorgt, andererseits aber auch die Möglichkeit eröffnet, durch das Ventil Instrumente oder Endoskope hindurchzuschieben. Hierzu sind unterschiedliche Konstruktionen an Ventilen mit Klappen oder mit Schlitzdichtungen bekannt.

Aufgrund der zuvor erwähnten weiten Verbreitung der minimal-invasiven Chirurgie wird eine Vielzahl an Zugangskanülen benötigt, die dann nach der Operation entsprechend gereinigt und sterilisiert werden müssen, was hochwertige Materialien erfordert.

Aus der eingangs erwähnten WO-A-01/08563 ist eine Zugangskanüle bekannt, die von der Innenseite eines menschlichen Körpers eingesetzt werden kann. Nach dem Setzen der Zugangskanüle kann ein mehrteiliger Dichtungskörper auf den aus dem Körper herausragenden Abschnitt der Kanüle montiert werden. Die Zugangskanüle weist ein Kanülenrohr auf, an dessen körperinnerem Ende fest ein Ventil angebracht ist, das ein Austreten von Flüssigkeiten oder Gasen aus dem Körperinneren verhindert. An dem anderen, vom Körper abstehenden Ende ist zusätzlich ein weiterer Ventilkörper montierbar. Dies jedoch erst, nachdem die Zugangskanüle mit dem festen Ventil bereits gesetzt ist.

Aus der US-A-5 800 451 ist eine Trokarhülse bekannt, die eine Zugangskanüle für endoskopische Operationen darstellt. Die Trokarhülse weist am proximalen Ende einen radial vorspringenden Ringflansch auf, um den ein Ventilkörper herum montiert werden kann. Dazu wird ein Teil eines Ventilgehäuses von distal nach proximal über die Trokarhülse geschoben, bis dieses Gehäuseteil an dem Flansch anstößt. Anschließend werden zahlreiche Einzelteile des Ventilsystems in das Gehäuse eingelegt und über seitlich bewegbare Klemmstangen zu einem mit der Zugangskanüle verbundenen Ventilkörper zusammengehalten. Zum Abnehmen des Ventilkörpers von dem einen Ende des Kanülenrohrs muß dies komplett zerlegt werden.

In der US-A-5 364 372 ist ein Trokar beschrieben, an dessen proximalem Ende ein mit dem Trokar fest verbundener Ventilkörper vorgesehen ist.

Eine ähnliche Konstruktion ist aus dem Dokument US-A-5 057 888 bekannt.

In der US-A-5 234 455 ist eine Zugangskanüle beschrieben, die am proximalen Ende fest mit einem Ventilkörper verbunden ist, in den auswechselbar eine Schlitzventilscheibe einsetzbar ist.

Es ist daher Aufgabe der vorliegenden Erfindung, eine Zugangskanüle für endoskopische Operationen zu schaffen, die unterschiedliche Operationsmethoden und unterschiedliche Möglichkeiten des Setzens der Kanüle ermöglicht und die kostengünstig herstellbar ist, dennoch aber effektiv und sicher während des Eingriffes arbeitet.

Erfindungsgemäß wird die Aufgabe dadurch gelöst, dass der Ventilkörper lösbar mit einem Ende des Kanülenrohrs verbindbar ist und das andere Ende des Rohrs offen ist, wobei der Ventilkörper einen Grundkörper mit einem Ansatz zum lösbaren Verbinden mit einem Ende des Kanülenrohrs aufweist und der Grundkörper zusammen mit dem Ventilkörper vom Kanülenrohr abnehmbar ist, und ferner ein Ventil aufweist, das für einen dichtenden Abschluß des Kanülenrohrs am Ende sorgt, jedoch ein Durchführen von Instrumenten durch das Kanülenrohr ermöglicht.

Es ist nun möglich, mit ein und demselben Ventilkörper verschiedene Kanülenrohre zu kombinieren, die für die jeweilige Operationstechnik am besten geeignet sind. Gleichzeitig eröffnet dies die Möglichkeit, zunächst einmal nur das Kanülenrohr zu setzen und erst später den Ventilkörper daran zu befestigen. Dies ermöglicht auch das Setzen von mehreren eng nebeneinander liegenden Kanülenrohren, die ja sehr schlank bauen, ohne daß zugleich der doch relativ voluminös bauende Ventilkörper vorhanden ist. Dadurch ist eine sehr hohe Flexibilität gegeben. Erst wenn die eigentliche Operation durchgeführt wird, also nach der Vorbereitung durch Setzen der Zugangskanüle, kann der Ventilkörper mit dem Kanülenrohr verbunden werden und damit für einen dichtenden Abschluß dieses Endes des Kanülenrohres sorgen. Durch den Ventilkörper selbst können dann die Instrumente, beispielsweise Endoskope oder Operationsinstrumente, hindurchgeschoben werden.

Eine erhöhte Flexibilität ist auch dadurch gegeben, daß an das Kanülenrohr auch andere Instrumente kurzfristig angesetzt werden können, wozu der Ventilkörper einfach abgenommen wird und ein Instrument, z.B. eine Beobachtungsoptik oder dergleichen, kurzzeitig angebracht werden kann.

Es ist also auch möglich, an einen einzigen Typ eines Ventils, das bestimmte Standardanschlüsse für andere Instrumente besitzt, unterschiedliche Kanülenrohre anzubringen, je nach Aufgabe und Operationstechnik.

Dies erhöht nicht nur die Flexibilität sondern sorgt auch für eine erhebliche Kostenreduzierung und stellt eine einwandfreie Abdichtung sicher. Die Art und Weise der lösbaren Verbindung zwischen Grundkörper und Kanülenrohr kann auf unterschiedliche Weisen erfolgen, z.B. durch Schraubverbindung, Bajonettverriegelung, Schnappverbindung etc.

In einer weiteren Ausgestaltung der Erfindung ist der Ventilkörper als sterilisierbares, mehrfach verwendbares Bauteil ausgebildet.

Diese Maßnahme hat den Vorteil, daß die zuvor erwähnte Zweigestaltung die Möglichkeit eröffnet, den Ventilkörper als hochwertiges Teil, beispielsweise aus Medizinstahl, herzustellen, das sterilisierbar und mehrfach verwendbar ist.

In einer weiteren Ausgestaltung der Erfindung ist das Kanülenrohr als Wegwerfbauteil ausgebildet.

Diese Maßnahme hat insbesondere mit der zuvor erwähnten Maßnahme den Vorteil, daß das Kanülenrohr als kostengünstiges Wegwerfteil für den einmaligen Gebrauch hergestellt werden kann, an das dann der wiederverwendbare Ventilkörper angekoppelt werden kann.

In einer weiteren Ausgestaltung der Erfindung ist das Ventil als Doppelscheibenventil ausgebildet.

Diese Maßnahme hat den Vorteil, daß durch die Doppelscheibenkonstruktion ein Instrument durch zwei Scheiben hindurchgeführt werden muß, was für eine besonders sichere Abdichtung sorgt.

In einer weiteren Ausgestaltung der Erfindung ist in jeder Scheibe eine Schlitzdichtung vorhanden.

Diese Maßnahme hat den Vorteil, daß Instrumente mit unterschiedlichem Durchmesser hindurchgeführt werden können, wobei jeweils ein dichter Abschluß gewährleistet ist. Dies ist insbesondere deswegen der Fall, da das Ventil als Doppelscheibenventil ausgebildet ist.

In einer weiteren Ausgestaltung der Erfindung sind die Schlitze sternförmig ausgebildet, und die Schlitze der beiden Scheiben sind gegeneinander verdreht.

Diese Maßnahme trägt noch weiter zusätzlich zu dem hervorragenden dichten Abschluß bei.

In einer weiteren Ausgestaltung der Erfindung sind die beiden Scheiben über einen elastischen Steg miteinander verbunden.

Diese Maßnahme hat den erheblichen Vorteil, daß das Doppelscheibenventil lediglich aus einem Bauteil besteht.

In einer weiteren Ausgestaltung der Erfindung sind an den Scheiben Rastmittel vorhanden, über die die Scheiben miteinander verrastbar sind.

Diese Maßnahme hat den Vorteil, daß das Doppelscheibenventil als sich in einer Ebene erstreckender Körper hergestellt werden kann, bei dem die beiden Scheiben über den Steg miteinander verbunden sind und zur Montage durch Verbiegen des Steges um- und aufeinander gelegt werden und dann durch die Rastmittel fest aneinander gehalten sind.

In einer weiteren Ausgestaltung der Erfindung ist der Ventilkörper zweiteilig ausgebildet, und zwischen den beiden Teilen ist das Ventil einbringbar.

Diese Maßnahme hat den Vorteil, daß durch konstruktiv einfache Maßnahmen das Ventil im Ventilkörper gehalten ist. Diese Maßnahme erlaubt auch ein einfaches Zerlegen des Ventilkörpers zum Reinigen und zum Sterilisieren.

In einer weiteren Ausgestaltung der Erfindung weist der Ventilkörper einen Grundkörper auf, der einen Ansatz zum lösbaren Verbinden mit dem Kanülenrohr aufweist, und der ferner eine Ausnehmung aufweist, in die das Ventil einlegbar ist, wobei das Ventil über einen Klemmring festlegbar ist.

Diese Maßnahme hat den Vorteil, daß ventilgrundkörper und Klemmring aus hochwertigen Edelstahlmaterialien hergestellt werden können, wohingegen das zwischengelegte Ventil durch ein Wegwerfteil ausgebildet werden kann. Daher müssen lediglich diese beiden Teile sterilisiert werden, nach dem Sterilisieren kann ein neues Ventil eingelegt werden.

Dies erhöht weiter die Flexibilität und trägt auch zur Kostenreduzierung bei. In diesem Fall kann dann sowohl das Kanülenrohr als auch das Ventil als Wegwerfteil ausgebildet sein und lediglich der zweiteilige Ventilkörper aus hochwertigen und präzis gefertigten Materialien hergestellt sein.

In einer weiteren Ausgestaltung der Erfindung weist das Kanülenrohr distalseitig einen nach proximal konisch aufweitenden Ring auf.

Diese Maßnahme hat den Vorteil, daß durch diesen Ring, insbesondere bei arthroskopischen Operationen, das Kanülenrohr in das Gelenk hineingeschoben bzw. hineingedrückt werden kann und gegen ein Abziehen oder versehentliches Lösen während der Operation gesichert ist, da ja die distale Spitze mit dem konischen Ring in das Gelenk quasi hineingeschnappt ist.

In einer weiteren Ausgestaltung der Erfindung ist das Kanülenrohr als Bauteil einer Trokarvorrichtung ausgebildet.

Diese Maßnahme hat den Vorteil, daß dadurch ein flexibler Bausatz an unterschiedlichen Trokaren möglich ist, beispielsweise für Erwachsene, für Kinder, für dünnleibige und für dickleibige Personen.

In einer weiteren Ausgestaltung der Erfindung weist die Trokarvorrichtung einen Dorn mit einem Handgriff samt Mandrin auf, wobei der Dorn samt Mandrin in das Kanülenrohr einführbar ist.

Diese Maßnahme hat den Vorteil, daß die Zugangskanüle quasi als Trokarhülse ausgebildet ist, die beim Setzen zusammen mit dem Dorn und dem Mandrin in den Körper eingeführt wird.

In einer weiteren Ausgestaltung der Erfindung ist das Kanülenrohr als Bauteil einer Dilatationsvorrichtung ausgebildet.

Diese Maßnahme hat nun den erheblichen Vorteil, daß bei einem Zugang eine Dilatation ohne Traumatisierung durchgeführt werden kann. Beispielsweise bei arthroskopischen Operationen wird zunächst mit sehr dünnen Kanülen ein erster Zugang bewerkstelligt, durch das Aufschieben von nach und nach immer durchmessergrößeren Dilatationsdornen wird dieser aufgeweitet, und letztendlich wird die erfindungsgemäße Zugangskanüle als im Körper verbleibende Kanüle für die Operation gesetzt.

Besonders vorteilhaft weist dazu die Dilatationsvorrichtung eine Punktionsnadel, einen Führungsdraht, zumindest einen Dilatationsdorn zum Aufsetzen auf den Führungsdraht auf, wobei das Kanülenrohr derart ausgebildet ist, daß es auf einen Dilatationsdorn aufschiebbar ist.

Diese Anordnung ist insbesondere bei dem atraumatischen Setzen des Kanülenrohrs für eine arthroskopische Operation von Vorteil, wobei sich hier diese Flexibilität besonders günstig auswirkt. Dabei kann das Führungsrohr wieder mit dem mit einem Handgriff versehenen Dorn auf den Dilatationsdorn aufgeschoben werden.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: einen Längsschnitt einer erfindungsgemäßen Zugangskanüle, bestehend aus Kanülenrohr und Ventilkörper, in Explosionsdarstellung;
- Fig. 2: einen um 90° um die Kanülenrohrachse verdrehten Schnitt durch den Ventilkörper;
- Fig. 3: eine perspektivische Ansicht des im Ventilkörper eingebauten Ventils;
- Fig. 4: das in Fig. 3 dargestellte Doppelscheibenventil in einem ausgeklappten Zustand vor Montage in dem Ventilkörper;
- Fig. 5: eine entsprechende um 180° gedrehte perspektivische Ansicht des Ventils;
- Fig. 6: einen der Fig. 1 entsprechenden Längsschnitt des Zusammenbaus aus Kanülenrohr und Ventilkörper;
- Fig. 7: einen Dorn mit Handgriff einer Trokarvorrichtung;
- Fig. 8: einen Mandrin einer Trokarvorrichtung, die mit einer erfindungsgemäßen Zugangskanüle von Fig. 6 zusammenwirken soll;
- Fig. 9: einen Zusammenbau der in Fig. 6, 7 und 8 dargestellten Bauteile;
- Fig. 10: eine Punktionsnadel einer Dilatationsvorrichtung;
- Fig. 11: ein Innenteil der Punktionsnadel von Fig. 10;
- Fig. 12: einen Führungsdraht, der in die Punktionsnadel von Fig. 10 einschiebbar ist;
- Fig. 13: einen Dilatationsdorn nebst Handgriff, der über den Führungsdraht von Fig. 12 schiebbar ist; und
- Fig. 14: einen Zusammenbau aus der Zugangskanüle von Fig. 1 bzw. 6 mit einem darin eingesetzten Dorn von Fig. 7, um über den Dilatationsdorn von Fig. 13 geschoben zu werden.

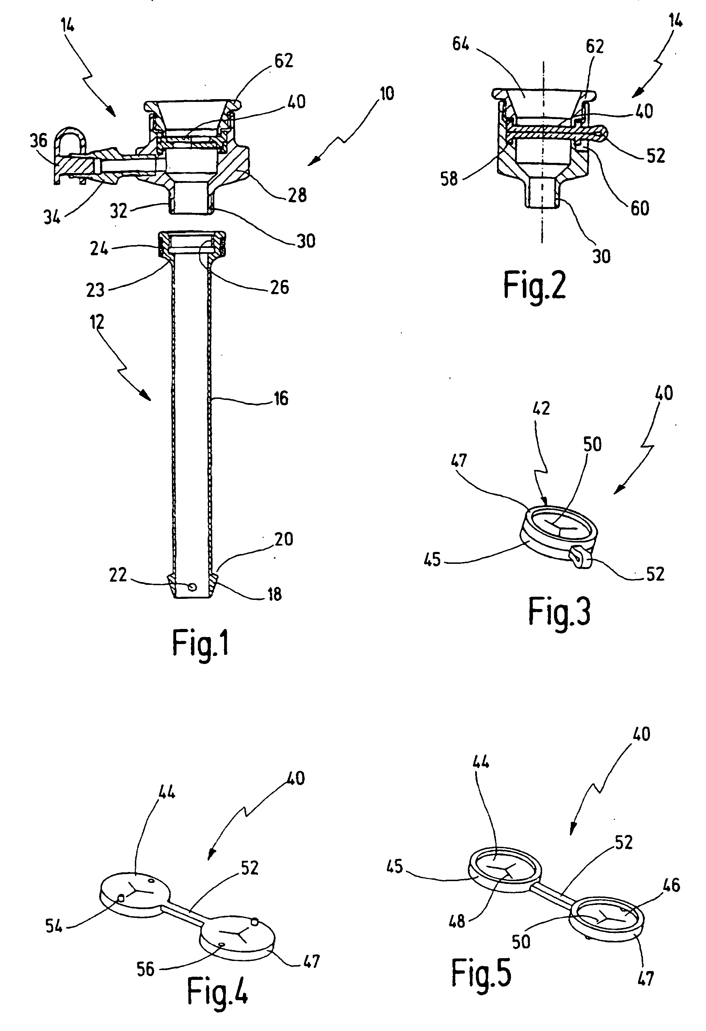

Eine in Fig. 1 dargestellte Zugangskanüle ist in ihrer Gesamtheit mit der Bezugsziffer 10 versehen.

Die Zugangskanüle 10 besteht aus einem Kanülenrohr 12 und einem Ventilkörper 14.

Das Kanülenrohr 12 weist ein Rohr 16 auf, an dessen distalem Ende ein konischer Ring 18 als Nase vorhanden ist. Die Konizität ist so gewählt, daß sich der Ring 18 von distal nach proximal gesehen erweitert.

Daher ist im Abstand vom distalen Ende eine Schulter 20 geschaffen. Dieser Ring 18 dient dazu, in eine Körperöffnung, beispielsweise in ein Gelenk, eingetrieben zu werden und dann darin einzurasten, um ein Abziehen des Kanülenrohrs 12 zu sperren.

Im Bereich des konischen Rings 18 ist seitlich in der Wand eine durchgehende Öffnung 22 vorgesehen, die dazu dient, einen Druckausgleich mit der Umgebung bei Operationen zu ermöglichen. An dem dem konischen Ring 18 gegenüberliegenden Ende 23 ist das Rohr 16 mit einem durchmessergrößeren Kragen 24 versehen, der ein Innengewinde 26 aufweist.

Der Ventilkörper 14 weist einen Grundkörper 28 auf, von dem von einer Seite ein rohrförmiger Ansatz 30 vorspringt, der mit einem Außengewinde 32 versehen ist. Das Außengewinde 32 ist so ausgebildet, daß es in das Innengewinde 26 im Kragen 24 des Rohres 16 eingedreht werden kann. Der lichte Innendurchmesser des Ansatzes 30 entspricht dem lichten Innendurchmesser des Rohres 16. Von dem Grundkörper 28 springt seitlich ein Stutzen 34 vor, der über eine Kappe 36 verschlossen ist. Dieser seitliche Stutzen 34 dient dazu, um Flüssigkeiten oder Gase seitlich über den Ventilkörper 14 dem Rohr 16 zuzuführen oder von diesem abzuleiten.

Im Ventilkörper 14 ist ein Ventil 40 angeordnet, und zwar proximalseitig des seitlichen Stutzens 34. Das Ventil 40 sorgt somit für einen dichten Abschluß des Ventilkörpers 14 nach proximal bzw. des Kanülenrohrs 12, wenn der Ventilkörper 14 mit diesem über die Schraubverbindung verbunden ist.

Das Ventil 40 besteht dabei aus einem Doppelscheibenventil 42, wie das insbesondere aus den Fig. 2 bis 5 ersichtlich ist.

Das Doppelscheibenventil 42 weist zwei Scheiben 44 und 46 auf, die jeweils von einem Ring 45 bzw. 47 umgeben sind. In jeder Scheibe 44 bzw. 46 ist ein dreistrahlig sternförmiger Schlitz 48 bzw. 50 vorgesehen. Das Material des Doppelscheibenventils besteht aus einem gummielastischen Kunststoffmaterial, so daß trotz Vorhandenseins der Schlitze 48 und 50 ein dichter Abschluß durch jede Scheibe 44 bzw. 46 gewährleistet ist.

Die beiden Ringe 45 und 47 sind über einen flexiblen Steg 52 miteinander verbunden. Aus den perspektivischen Darstellungen von Fig. 4 und 5 ist ersichtlich, daß die Schlitze 48 und 50 jeweils verdreht zueinander eingeschnitten sind.

Wie insbesondere aus Fig. 4 ersichtlich, springt von jeder Scheibe ein Zapfen 54 vor, der in eine entsprechende Öffnung 56 an der anderen Scheibe einsteckbar ist.

Das Doppelscheibenventil 42 ist somit, in der Ausrichtung, wie es in Fig. 4 und 5 dargestellt ist, als Form- oder Stanzteil herstellbar, und anschließend wird es durch Umbiegen um den Steg 52 in die in Fig. 3 dargestellte Stellung gebracht.

Das Ventil 40 wird in eine Ausnehmung 58 im Grundkörper 28 des Ventilkörpers 14 eingelegt, wobei dieser eine seitliche Schlitzöffnung 60 aufweist, aus der der umgelegte Steg 52 seitlich herausreichen kann. Das Ventil 40 ist über einen Klemmring 62 am Ventilkörper 14 gehalten, der dazu mit einem Außengewinde versehen ist, das in ein hier nicht näher gezeigtes Innengewinde in der Ausnehmung 58 eingedreht werden kann. Der Klemmring 62 drückt dann die beiden Ringe 45 und 47 der Scheiben 44 und 46 dichtend aufeinander, so daß insgesamt gesehen durch das Ventil 40 ein dichter Abschluß des Ventilkörpers 14 gewährleistet ist.

Im Innern des Ventilkörpers 14 ist eine durchgehende Öffnung 64 vorgesehen, deren lichter Innendurchmesser größer ist als der lichte Innendurchmesser des Rohres 16, so daß dann beispielsweise am Ventilkörper 14 Instrumente, wie z.B. Endoskope oder dergleichen, angesetzt werden können.

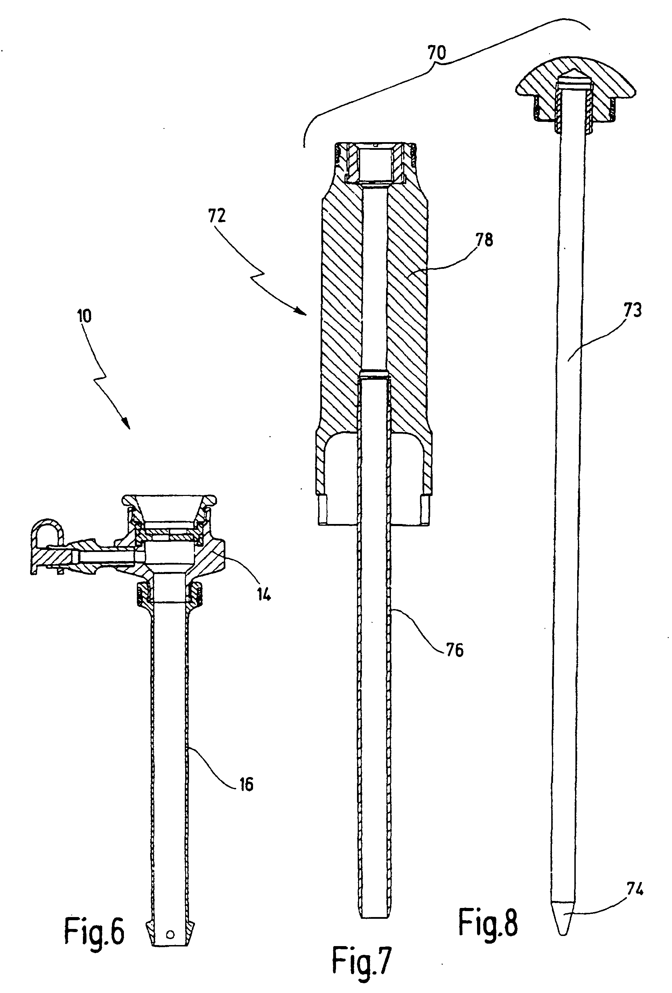

In Fig. 6 ist die Zugangskanüle 10 im zusammengebauten Zustand dargestellt, d.h. der Ventilkörper 14 ist endfertig montiert, d.h. das Ventil 40 ist eingesetzt, und der Ventilkörper 14 ist über dessen Außengewinde 32 am Ansatz 30 in das Innengewinde 26 am Kragen 24 des Kanülenrohrs 12 eingedreht.

In diesem Zustand kann nun beispielsweise die Zugangskanüle 10 Bauteil oder Bestandteil einer Trokarvorrichtung 70 sein, wie sie in den Fig. 7 und 8 dargestellt ist.

Die Trokarvorrichtung 70 weist also einen Dorn 72 und einen Mandrin 73 auf, der eine angeschärfte Spitze 74 aufweist.

Der Dorn 72 weist einen Handgriff 78 auf, von dem eine rohrförmige Hülse 76 vorspringt.

Der Außendurchmesser der Hülse 76 ist so gewählt, daß diese dem lichten Innendurchmesser des Rohrs 16 der Zugangskanüle 10 entspricht.

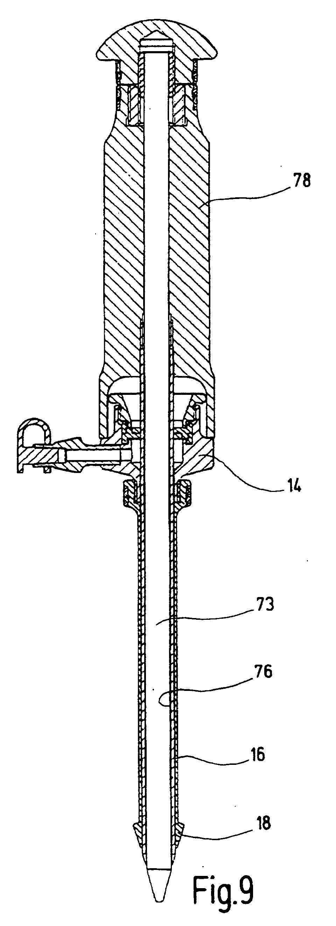

Die Trokarvorrichtung 70 ist in ihrem zusammengebauten Zustand in Fig. 9 dargestellt, d.h. der Mandrin 73 ist in die Hülse 76 des Dorns 72 eingeschoben, und dieser Zusammenbau ist wiederum in das Kanülenrohr 12 eingeschoben.

Aus der Darstellung von Fig. 9 ist zu erkennen, daß die Spitze 74 die Hülse 76 etwas überragt, diese überragt wiederum den konischen Ring 18 und ist mit einer entsprechenden konischen Fase versehen. Dadurch entsteht ein spitzkegeliges Eintreibende der Trokarvorrichtung 70.

Beim Setzen der Zugangskanüle 10, die nun als Trokarhülse fungiert, wird der Mandrin 73 an einer Hautincision angesetzt und in den Körper eingestochen, und der gesamte Zusammenbau von Fig. 9 wird bis zur gewünschten Tiefe in den Körper eingetrieben. Der Handgriff 78 erleichtert dabei diesen Vorgang. Anschließend werden Dorn 72 und Mandrin 73 abgezogen. Aufgrund des Ventilkörpers 14 mit dem geschlossenen Ventil 40 ist dabei ausgeschlossen, daß Körperflüssigkeiten oder dergleichen austreten.

Es ist auch möglich, das Kanülenrohr 12 zunächst einmal ohne den Ventilkörper 14 in den Körper einzusetzen, und erst anschließend den Ventilkörper 14 aufzuschrauben.

Es ist auch möglich, wie zuvor beschrieben den Zusammenbau, wie in Fig. 6 gezeigt, einzutreiben und nach Abziehen von Dorn 72 und Mandrin 73, wenn notwendig, den Ventilkörper 14 kurzzeitig abzudrehen, um beispielsweise in unmittelbarer Nähe eine zweite Zugangskanüle zu setzen.

Dadurch wird die Flexibilität besonders deutlich.

Nach Abziehen der Zugangskanüle 10 kann der Ventilkörper 14 zerlegt, gereinigt und sterilisiert werden, wobei je nach Ausgestaltung das Doppelscheibenventil 42 ebenfalls gereinigt und wieder eingesetzt werden kann oder durch ein anderes ersetzt werden kann.

Je nach Ausgestaltung kann auch das Kanülenrohr 12 gereinigt und sterilisiert werden, oder es kann auch als Wegwerfteil ausgebildet sein und nach der Operation verworfen werden. Dann kann der gereinigte, sterilisierte und wieder zusammengesetzte Ventilkörper 14 mit einem Kanülenrohr 12 erneut verbunden werden.

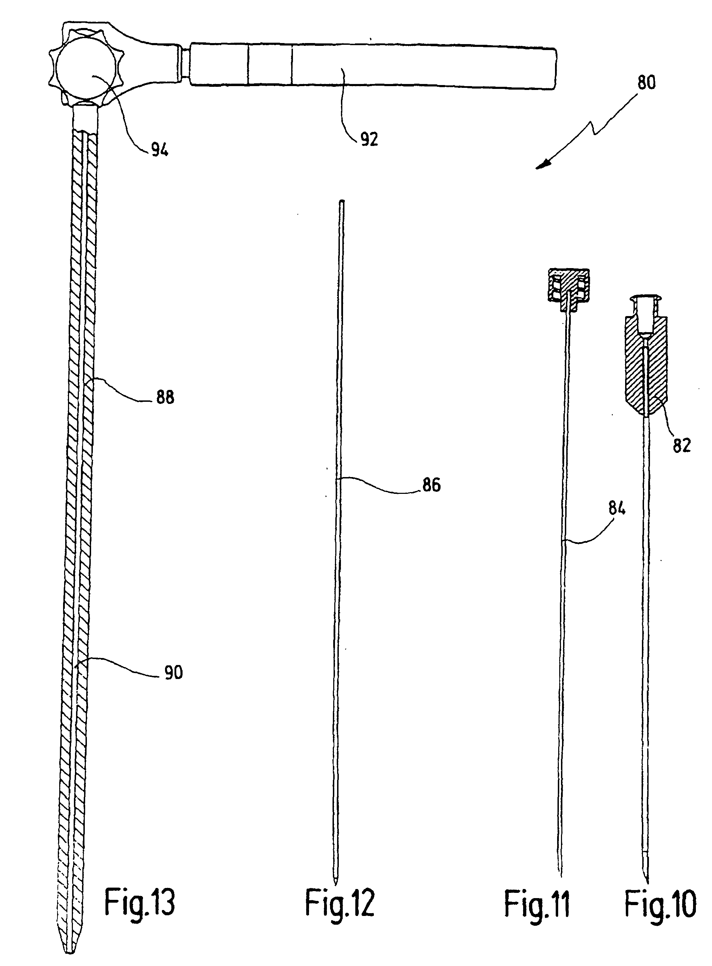

In den Fig. 10 bis 14 ist eine weitere Einsatzmöglichkeit der erfindungsgemäßen Zugangskanüle 10 dargestellt, wobei es sich hier um einen Zugang für eine arthroskopische Operation, beispielsweise eine Operation an einem Schultergelenk, handelt. Hierzu wird die in Fig. 10 dargestellte Punktionsnadel 82 an der Stelle angesetzt, in der zwischen zwei Gelenkknochen ein Zugang gewünscht wird. Der Durchmesser der Punktionsnadel 82 ist äußerst gering und liegt im Bereich von etwa 1,5 mm.

Die Nadel wird durch die Haut bis in das Gelenk vorgeschoben, bis die Nadelspitze durch ein Arthroskop erkannt werden kann.

In der Punktionsnadel 82, die als Hohlnadel ausgebildet ist, steckt ein Innenteil 84.

Nach Einführen der Punktionsnadel 82 in das Gelenk wird das Innenteil 84 abgenommen und ein Führungsdraht 86 in die Punktionsnadel 82 vollständig eingeschoben.

Anschließend wird die Punktionsnadel 82 abgezogen, und der Führungsdraht 84 steckt nun im Körper bzw. im Gelenk.

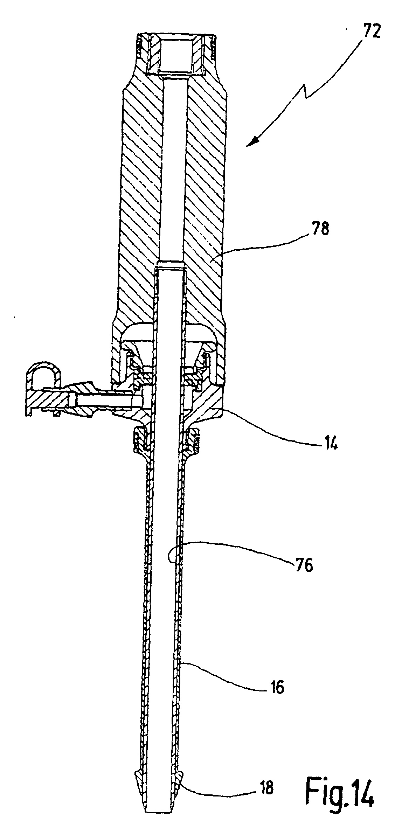

Nach Durchführen einer Hautincision wird auf den Führungsdraht 86 ein Dilatationsdorn 88 aufgeschoben, der dazu, wie in Fig. 13 dargestellt ist, mit einem Handgriff 92 verbunden ist. Die Verbindung zwischen Handgriff 92 und Dilatationsdorn 88 erfolgt über eine Feststellschraube 94. Je nachdem, auf welchen Durchmesser erweitert werden soll, werden ein oder mehrere immer durchmessergrößere Dilatationsdorne 88 aufgeschoben.

Im dargestellten Ausführungsbeispiel wird zunächst lediglich der Dilatationsdorn 88 auf den Führungsdraht 86 aufgeschoben und in das Gelenk hineingedrückt, wobei dies durch die konische Spitze des Dilatationsdorns 88 erleichtert wird.

Der Außendurchmesser des Dilatationsdornes 88 entspricht dabei dem lichten Innendurchmesser einer Hülse 76 eines Dorns 72, der wiederum in das Rohr 16 einer erfindungsgemäßen Zugangskanüle 10 eingeschoben ist.

Dieser Zusammenbau ist in Fig. 14 dargestellt.

Nach Setzen des Dilatationsdornes 88 wird der Handgriff 92 durch Lösen der Feststellschraube 94 abgenommen, und über den Dilatationsdorn 88 wird der Zusammenbau von Fig. 14 aufgeschoben. Dabei wird die Zugangskanüle 10 wieder so weit eingeschoben, bis deren konischer Ring 18 in das Gelenk eingeschnappt bzw. eingerastet ist. Dabei müssen erhebliche Kräfte aufgebracht werden, wobei der Handgriff 78 insbesondere wegen dessen griffiger Form dies auf sichere Art und Weise ermöglicht.

Anschließend wird der Dilatationsdorn 88 und der Dorn 72 abgezogen, und lediglich die in Fig. 6 dargestellte Zugangskanüle 10 verbleibt im Körper. Anschließend kann dann der eigentliche operative Eingriff durch Hindurchführen der entsprechenden Instrumente durchgeführt werden.

## Patentansprüche

1. Zugangskanüle für endoskopische Operationen, insbesondere für die Arthroskopie, mit einem Kanülenrohr (12) und mit einem Ventilkörper (14), der lösbar mit einem Ende (23) des Kanülenrohrs (12) verbindbar ist, wobei das andere Ende des Rohrs offen ist, wobei der Ventilkörper (14) einen Grundkörper (28) mit einem Ansatz (30) zum lösbaren Verbinden mit einem Ende (23) des Kanülenrohrs (12) aufweist und ferner ein Ventil (40) aufweist, das für einen dichtenden Abschluß des Kanülenrohrs (12) am Ende (23) sorgt, jedoch ein Durchführen von Instrumenten durch das Kanülenrohr (12) ermöglicht **dadurch gekennzeichnet, dass** der Grundkörper (28) zusammen mit dem Ventilkörper (14) vom Kanülenrohr (12) abnehmbar ist.

2. Zugangskanüle nach Anspruch 1, **dadurch gekennzeichnet, daß** der Ventilkörper (14) als sterilisierbares, mehrfach verwendbares Bauteil ausgebildet ist.

3. Zugangskanüle nach Anspruch 1 oder 2, **dadurch gekennzeichnet, daß** das Kanülenrohr (12) als Wegwerfbauteil ausgebildet ist.

4. Zugangskanüle nach einem der Ansprüche 1 bis 3, **dadurch gekennzeichnet, daß** das Ventil (40) als Doppelscheibenventil (42) ausgebildet ist.

5. Zugangskanüle nach Anspruch 4, **dadurch gekennzeichnet, daß** in jeder Scheibe (44, 46) des Doppelscheibenventils (42) eine Schlitzdichtung vorhanden ist.

6. Zugangskanüle nach Anspruch 5, **dadurch gekennzeichnet, daß** Schlitze (48, 50) der Schlitzdichtung sternförmig ausgebildet sind, und daß die Schlitze (48, 50) der beiden Scheiben (44, 46) des Doppelscheibenventils (42) gegeneinander verdreht sind.

7. Zugangskanüle nach Anspruch 5 oder 6, **dadurch gekennzeichnet, daß** die beiden Scheiben (44, 46) über einen elastischen Steg (52) miteinander verbunden sind.

8. Zugangskanüle nach einem der Ansprüche 5 bis 7, **dadurch gekennzeichnet, daß** an den Scheiben (44, 46) Rastmittel vorhanden sind, über die die Scheiben (44, 46) miteinander verrastbar sind.

9. Zugangskanüle nach einem der Ansprüche 1 bis 8, **dadurch gekennzeichnet, daß** der Ventilkörper (14) zweiteilig ausgebildet ist, und daß zwischen den beiden Teilen (28, 62) das Ventil (40) anbringbar ist.

10. Zugangskanüle nach Anspruch 9, **dadurch gekennzeichnet, daß** der Ventilkörper (14) ferner eine Ausnehmung (58) aufweist, in die das Ventil (40) einlegbar ist, wobei das Ventil (40) über einen Klemmring (62) festlegbar ist.

11. Zugangskanüle nach einem der Ansprüche 1 bis 10, **dadurch gekennzeichnet, daß** das Kanülenrohr (12) distalseitig einen nach proximal konisch aufweitenden Ring (18) aufweist.

12. Zugangskanüle nach einem der Ansprüche 1 bis 11, **dadurch gekennzeichnet, daß** das Kanülenrohr (12) als Bauteil einer Trokarvorrichtung (70) ausgebildet ist.

13. Zugangskanüle nach Anspruch 12, **dadurch gekennzeichnet, daß** die Trokarvorrichtung (70) einen Dorn (72) mit einem Handgriff (78) samt Mandrin (73) aufweist, wobei der Dorn (72) in das Kanülenrohr (12) einführbar ist.

14. Dilatationsvorrichtung mit einem Kanülenrohr, **dadurch gekennzeichnet, daß** das Kanülenrohr als Zugangskanüle nach einem der Ansprüche 1 bis 11 ausgebildet ist.

15. Dilatationsvorrichtung nach Anspruch 14, **dadurch gekennzeichnet, daß** diese eine Punktionsnadel (82), einen Führungsdraht (86) und zumindest einen Dilatationsdorn (88) zum Aufsetzen auf den Führungsdraht (86) aufweist, wobei das Kanülenrohr (12) derart ausgebildet ist, daß es auf einen Dilatationsdorn (88) aufschiebbar ist.

16. Dilatationsvorrichtung nach Anspruch 15, **dadurch gekennzeichnet, daß** das Kanülenrohr (12) mittels eines mit einem Handgriff (78) versehenen Dorn (72) auf den Dilatationsdorn (88) aufschiebbar ist.

## Claims

1. Access cannula for endoscopic surgeries, in particular for arthroscopy, with a cannula tube (12) and with a valve body (14), which can be connected releasably to one end (23) of the cannula tube (12), wherein the other end of that tube is open, said valve body (14) having a main body (28) with a neck (30) for releasably connecting one end (23) of the cannula tube (12) and having a valve (40) which insures a tight closure of the cannula tube (12) at the end (23), but allows instruments to be passed through the cannula tube (12), **characterized in that** said main body (28) is releasable from the cannula tube (12) together with the valve body (14).

2. Access cannula of claim 1, **characterized in that** the valve body (14) is designed as a sterilizable component part which can be used a number of times.

3. Access cannula of claim 1 or 2, **characterized in that** the cannula tube (12) is designed as a disposable component part.

4. Access cannula of anyone of claims 1 through 3, **characterized in that** the valve (40) is designed as a double-disk valve (42).

5. Access cannula of claim 4, **characterized in that** a slit seal is provided in each disk (44, 46) of the double-disk valve (42).

6. Access cannula of claim 5, **characterized in that** the slits (48, 50) of the slit seal are designed in a star shape, and **in that** the slits (48, 50) of the two disks (44, 46) of the double-disk valve (42) are offset in relation to one another.

7. Access cannula of claims 5 or 6, **characterized in that** the two disks (44, 46) are connected to one another via an elastic bridge (52).

8. Access cannula of anyone of claims 5 through 7, **characterized in that** locking means are provided on the disks (44, 46) and serve to lock the disks (44, 46) onto one another.

9. Access cannula of anyone of claims 1 through 8, **characterized in that** the valve body (14) is designed in two parts, and **in that** the valve (40) can be arranged between the two parts (28, 62).

10. Access cannula of claim 9, **characterized in that** the valve body (14) moreover has a recess (58) into which the valve (14) can be inserted, wherein said valve (40) being fixed via a clamping ring (62).

11. Access cannula of anyone of claims 1 through 10, **characterized in that** the cannula tube (12) has, at the distal end, a ring (18) which widens conically in the proximal direction.

12. Access cannula of anyone of claims 1 through 11, **characterized in that** the cannula tube (12) is designed as a component part of a trocar device (70).

13. Access cannula of claim 12, **characterized in that** the trocar device (70) has a mandrel (72) with a hand grip (78) and also a core rod (73), which mandrel (72) can be introduced into the cannula tube (12).

14. Dilatation device comprising a cannula tube, **characterized in that** the cannula tube is designed as an access cannula according to anyone of claims 1 through 11.

15. Dilatation device of claim 14, **characterized by** a puncture needle (82), a guide wire (86) and at least one dilatation mandrel (88) for placing onto the guide wire (86), said cannula tube (12) is designed in that it can be pushed onto a dilatation mandrel (88).

16. Dilatation device of claim 15, **characterized in that** said cannula tube (12) can be pushed onto the dilatation mandrel (88) via a hand grip (78) having a mandrel (72).

## Revendications

1. Canule d'accès pour opérations endoscopiques, en particulier pour l'arthroscopie, avec un tube de canule (12) et un élément formant valve (14) qui peut être relié, de manière détachable, à une extrémité (23) du tube de canule (12), l'autre extrémité du tube étant ouverte, l'élément formant valve (14) comportant un corps de base (28) avec un embout (30) pour la liaison détachable à une extrémité (23) du tube de canule (12) et, en outre, une valve (40) qui assure que la terminaison du tube de canule (12) soit étanche à l'extrémité (23) mais permet l'introduction d'instruments à travers le tube de canule (12), **caractérisée en ce que** le corps de base (28) peut être retiré du tube de canule (12) avec l'élément formant valve (14).

2. Canule d'accès selon la revendication 1, **caractérisée en ce que** l'élément formant valve (14) est configuré en tant que composant réutilisable, stérilisable.

3. Canule d'accès selon la revendication 1 ou 2, **caractérisée en ce que** le tube de canule (12) est configuré en tant que composant jetable.

4. Canule d'accès selon l'une des revendications 1 à 3, **caractérisée en ce que** la valve (40) est configurée en tant que valve à double disque (42).

5. Canule d'accès selon la revendication 4, **caractérisée en ce que** dans chaque disque (44, 46) de la valve à double disque (42) se trouve une garniture d'étanchéité à fentes.

6. Canule d'accès selon la revendication 5, **caractérisée en ce que** les fentes (48, 50) de la garniture d'étanchéité sont configurées en étoile et que les fentes (48, 50) des deux disques (44, 46) de la valve à double disque (42) sont décalées les unes par rapport aux autres.

7. Canule d'accès selon la revendication 5 ou 6, **caractérisée en ce que** les deux disques (44, 46) sont reliés l'un à l'autre par le biais d'une tige élastique (52).

8. Canule d'accès selon l'une des revendications 5 à 7, **caractérisée en ce que**, au niveau des disques (44, 46), se trouvent des dispositifs d'accrochage par le biais desquels les disques (44, 46) peuvent être encliquetés l'un dans l'autre.

9. Canule d'accès selon l'une des revendications 1 à 8, **caractérisée en ce que** l'élément formant valve (14) est configuré en deux parties et que la valve (40) peut être montée entre les deux parties (28, 62).

10. Canule d'accès selon la revendication 9, **caractérisée en ce que** l'élément formant valve (14) comporte en outre un évidement (58) dans lequel il est possible d'insérer la valve (40), cette dernière pouvant être fixée par le biais d'une bague de serrage (62).

11. Canule d'accès selon l'une des revendications 1 à 10, **caractérisée en ce que** le tube de canule (12) comporte, du côté distal, une bague (18) s'élargissant de manière conique en direction du côté proximal.

12. Canule d'accès selon l'une des revendications 1 à 11, **caractérisée en ce que** le tube de canule (12) est configuré en tant que composant d'un dispositif à trocart (70).

13. Canule d'accès selon la revendication 12, **caractérisée en ce que** le dispositif à trocart (70) comprend une broche (72) avec un manche (78), y compris un mandrin (73), la broche (72) pouvant être insérée dans le tube de canule (12).

14. Dispositif de dilatation avec un tube de canule, **caractérisé en ce que** le tube de canule est configuré en tant que canule d'accès selon l'une des revendications 1 à 11.

15. Dispositif de dilatation selon la revendication 14, **caractérisé en ce que** celui-ci comprend une aiguille à ponction (82), un fil guide (86) et au moins une broche de dilatation (88) à déposer sur le fil guide (86), le tube de canule (12) étant configuré de telle sorte qu'il puisse être enfilé sur une broche de dilatation (88).

16. Dispositif de dilatation selon la revendication 15, **caractérisé en ce que** le tube de canule (12) peut être enfilé, au moyen d'une broche (72) munie d'un manche (78), sur la broche de dilatation (88).
